# EUROPÄISCHE PATENTANMELDUNG

(11) **EP 1 386 631 A1**
(43) Veröffentlichungstag der Anmeldung: **04.02.2004**
(21) Anmeldenummer: 02017388.6
(22) Anmeldetag: 02.08.2002
(51) Int. Cl.: A61M 16/12, A61M 16/20, A61M 11/00

(54) **Vorrichtung zur Erzeugung eines kontinuierlichen positiven Atemwegdrucks (CPAP-Vorrichtung)**

(71) Anmelder: MED IN Medical Innovations Vetriebs GmbH, 81245 München (DE)
(72) Erfinder: Kniewasser, Gert, 82269 Kaltenberg (DE)
(74) Vertreter: Barth, Stephan Manuel, Dr.

(57) **Zusammenfassung**

Die vorliegende Erfindung schafft eine Vorrichtung zur Erzeugung eines kontinuierlichen positiven Atemwegdrucks (CPAP-Vorrichtung), insbesondere nasale CPAP-Vorrichtung (nCPAP-Vorrichtung), mit einem Hohlkörper (10), in dem ein Überdruck aufbaubar ist; einer in einer ersten Seitenwand des Hohlkörpers (10) vorgesehenen ersten Öffnung (20) zum Zuführen einer in den Hohlkörper (10) gerichteten Atemgasströmung (A) und zum Abführen der ausgeatmeten Atemgasströmung (B); und einem am Hohlkörper (10) anbringbaren Anschlussstück (30) zur Verbindung des Hohlkörpers (10) mit einem Nasen- und/oder Mundansatzstück (100). Es ist eine zweite Öffnung (71) zum Zuführen einer in den Hohlkörper (10) gerichteten Medikamentenströmung (M) in einer zweiten Seitenwand des Hohlkörpers (10) vorgesehen.

## Beschreibung

### STAND DER TECHNIK

Die vorliegende Erfindung betrifft eine Vorrichtung zur Erzeugung eines kontinuierlichen positiven Atemwegdrucks (CPAP-Vorrichtung), und insbesondere eine nasale CPAP-Vorrichtung oder kurz nCPAP-Vorrichtung, gemäss dem Oberbegriff des Anspruches 1, wie aus der WO 99/24101 bekannt.

Das Prinzip dieser CPAP- bzw. nCPAP-Vorrichtungen besteht darin, dass der Patient gegen einen Druck atmet, der höher als der Atmosphärendruck liegt, was die spontane Atmung in bestimmten Fällen behinderter Pulmonarfunktion erleichtert und verbessert.

Eine äusserst wichtige Anwendung liegt in der Neugeborenenbehandlung. Der Effekt dabei ist, dass die noch nicht ausgereifte Lunge durch den Überdruck aufgebläht wird und dadurch ein besserer Gasaustausch möglich ist. Ein weiterer sehr bedeutender Faktor ist, dass das gefährliche Kollabieren der Alveolen durch den Atmungsunterdruck, durch diese Technik verhindert werden kann. Noch ein Gesichtspunkt ist, dass die Alveolen besser geöffnet werden, und somit der gesamte Funktionszustand der Lunge verbessert wird.

Die EP-A-0 658 356 offenbart eine CPAP-Vorrichtung mit einem Paar von Nasenansatzstücken jeweils mit einer Kanülenspitze zum Einsetzen in die Nasenlöcher des Patienten.

Aus der DE-C-3 119 814 ist eine CPAP- bzw. nCPAP-Vorrichtung mit einem Atmungskanal, der sich an seinem freien Ende zur Atmosphäre hin öffnet und der an seinem anderen Ende mit einem Kupplungsstück zum Anbringen an der Nase und/oder dem Mund des Patienten versehen werden kann, und mit einem Einlasskanal für Frischgas, der mit dem Atmungskanal an einer Stelle zwischen dessen Enden verbunden ist und dessen Durchfluss einstellbar ist, bekannt.

Die EP 0 447 443 B1 offenbart eine Weiterbildung dieser bekannten CPAP- bzw. nCPAP-Vorrichtung, wobei der Atmungskanal einen ersten Abzweigkanal, der an dem Kupplungsstück anschliessbar ist, und einen zweiten Abzweigkanal, der zur Atmosphäre hin offen ist, aufweist. Die beiden Atmungskanäle bilden einen Winkel miteinander, so dass der Einlasskanal im wesentlichen in der Verlängerung des ersten Abzweigkanals liegt und mit dem zweiten Abzweigkanal so verbunden ist, dass der Frischgasstrom hauptsächlich koaxial in der ersten Abzweigkanal gerichtet ist und dadurch einen Ejektionsvorgang bewirkt. Die Querschnittsfläche des jeweiligen Abzweigkanals ist um ein Vielfaches grösser als die kleinste Querschnittsfläche des Einlasskanals, und die Länge jedes Abzweigkanals ist relativ kurz und beträgt vorzugsweise das Fünffache seines Innendurchmessers. Der Atmungskanal ist mit dem Einlasskanal als kompakte Einheit zusammengebaut, die an der Nase und/oder dem Mund des Patienten mittels eines Streifens oder entsprechenden Mittels anbringbar ist.

Fig. 3 zeigt eine schematische Darstellung einer aus der WO 99/24101 bekannten CPAP-Vorrichtung in perspektivischer Ansicht.

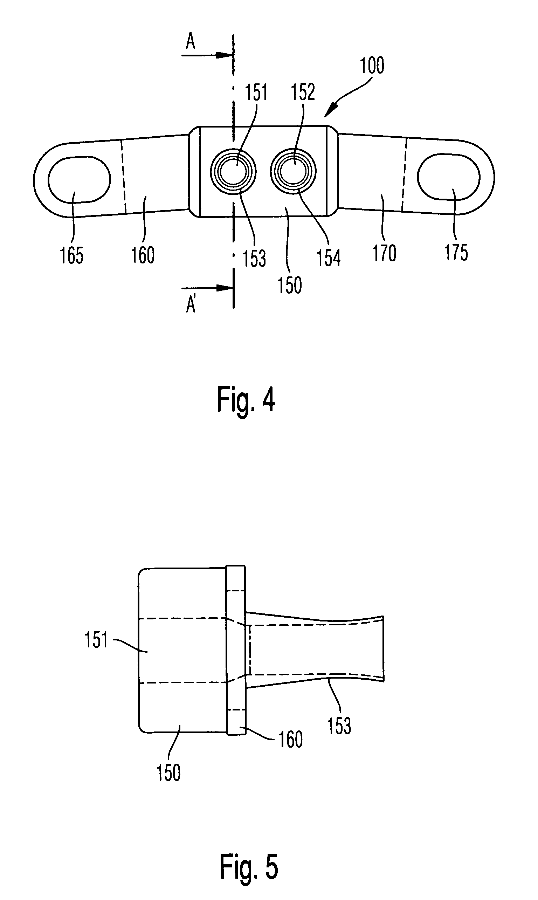

Hauptbestandteile dieser bekannten Vorrichtung zur Erzeugung eines kontinuierlichen positiven Atemwegdrucks sind ein zylindrischer Hohlkörper 10, in dem ein Überdruck aufbaubar ist, eine in der Mantelfläche des zylindrischen Hohlkörpers 10 vorgesehene erste Öffnung 20 zum Zuführen einer in den Hohlkörper 10 gerichteten Atemgasströmung A und zum Abführen der ausgeatmeten Atemgasströmung B, ein am zylindrischen Hohlkörper 10 an dessen einer Stirnfläche 10a anbringbares Anschlussstück 30 zur Verbindung des Hohlkörpers 10 mit einem Nasen- und/oder Mundansatzstück 100, das näher in Fig. 4 und 5 erläutert ist, und ein am Hohlkörper 10 anbringbarer Abstandshalter 40, an dem eine Strömungsdüse 50 zum Richten der Atemgasströmung A auf die Öffnung 20 anbringbar ist.

Das Anschlussstück 30 besteht aus einem Stopfen aus Teflon, der an der Stirnfläche 10a in den Hohlkörper 10 zumindest teilweise einführbar ist. Dieser Stopfen ist zweckmässigerweise ein abgestuftes Drehteil. Er weist zwei Durchführungen auf (gestrichelte Linien), welche entsprechenden Atem-Atemgaswegen des Nasenansatzstücks 100 entsprechen. In den Durchführungen des Anschlussstücks 30 sind zwei nach aussen weisende Röhrenstummel 35 zum Einführen in das Nasenund/oder Mundansatzstück 100 vorgesehen.

Durch seinen Aufbau als Stopfen ist das Anschlussstück 30 relativ zum Hohlkörper 10 um die Zylinderachse verdrehbar, die im wesentlichen senkrecht zur Achse der in den Hohlkörper 10 gerichteten Atemgasströmung A liegt.

An der anderen Stirnfläche 10b des zylindrischen Hohlkörpers 10 ist eine zweite Öffnung zum Anschliessen eines Druckmessgeräts vorgesehen. Dazu ist mit der zweiten Öffnung unter einem im wesentlichen rechtem Winkel eine Röhre 60 zum Anschluss an eine Druckmessleitung unter Verwendung einer im Hohlkörper 10 in der Stirnfläche 10b angebrachten Verstärkungsplatte (nicht dargestellt) verbunden.

Die Röhre 60 erstreckt sich im wesentlichen um die gleiche Länge vom Hohlkörper 10 weg wie die Strömungsdüse 50, so dass eine Leitung zur Zuführung von Druckatemgas an die Strömungsdüse im wesentlichen auf gleicher Höhe wie die Druckmessleitung an die Strömungsdüse 50 anbringbar ist.

Beim gezeigten Ausführungsbeispiel sind der Hohlkörper 10, der Abstandshalter 40, die Strömungsdüse 50, die Röhre 60, die Verstärkungsplatte und die Röhrenstummel 35 aus Aluminium oder Feinaluminium hergestellt.

Dabei ist der Abstandshalter 40 fest am Hohlkörper 10 durch Verbördeln mit einem eingesetzten Ring angebracht. Ausserdem ist die Strömungsdüse 50 fest am Abstandshalter 40 durch Verschrauben angebracht. Schliesslich ist die Röhre 60 fest und dicht am Hohlkörper 10 durch Verbördeln oder Verschrauben angebracht.

Alternativ dazu könnte die Röhre 60 relativ zum Hohlkörper 10 um mindestens eine bestimmte Achse verdrehbar angebracht sein, wobei auf eine entsprechende Dichtigkeit wie beim Anschlussstück 30 zu achten ist.

Der Abstandshalter 40 weist eine im wesentlichen ringförmige Gestalt auf. Die Strömungsdüse 50 ist durch ein Loch in der Seitenwand der Ringgestalt geführt und im wesentlichen senkrecht auf die Öffnung 20 gerichtet. Die Strömungsdüse 50 ragt um eine vorbestimmte Länge in das Ringgestaltinnere hinein.

Die Seitenwand der Ringgestalt weist eine Sicherheits-Atemgasdurchtrittsöffnung 45 auf. Die Ringgestalt dieser Konstruktion ist mit den Fingern derart verschliessbar, dass die ausgeatmete Atemgasströmung B durch die Sicherheits-Atemgasdurchtrittsöffnung 45 verläuft.

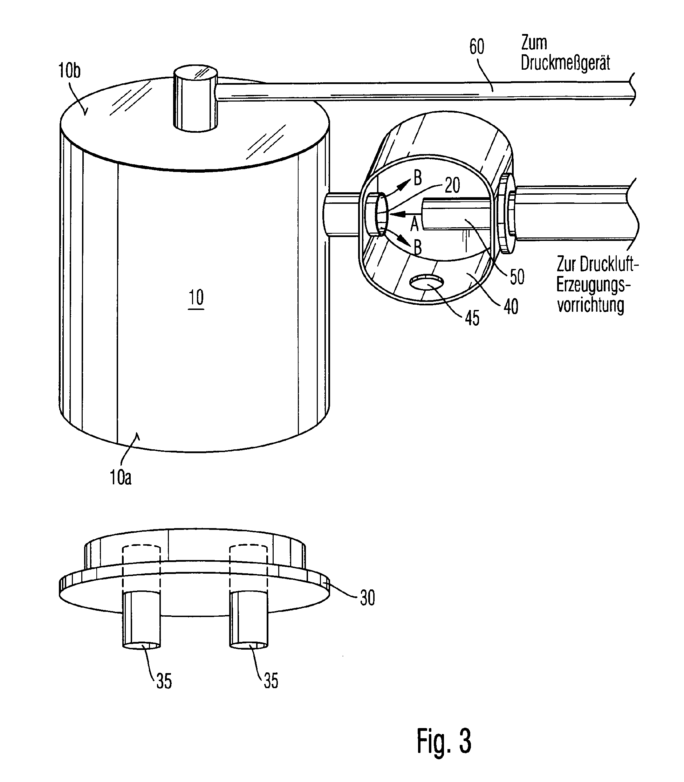

Fig. 4 zeigt eine erste Querschnittsansicht eines Nasenansatzstücks zum Anschluss an das Ansatzstück von Fig. 3.

Das gezeigte Nasenansatzstück 100 ist aus sehr weichem Silikon hergestellt. Es weist einen zentralen Teil 150 in Form eines Quaders mit zwei Löchern 151, 152 und zwei davon ausgehenden, aus der Zeichenebene nach oben herausragenden, dünnwandigen Naseneinführröhrchen 153, 154 auf. Auf beiden Seiten des Quaders sind Laschen 160, 170 angebracht, welche am Kopf des Patienten mittels eines Bandes oder entsprechenden Mittels anbringbar sind, das durch entsprechende Löcher 165, 175 führbar ist.

Fig. 5 zeigt eine zweite Querschnittsansicht des Ausführungsbeispiels eines Nasenansatzstücks zum Anschluss an das Ansatzstück entlang der Linie A-A', die in Fig. 4 angedeutet ist.

Im übrigen ist das Nasenansatzstück 100 zweckmässigerweise ein Wegwerfteil, da Silikon beim Autoklavieren aushärtet und damit die Naseneingänge des Patienten beschädigen könnte.

Im folgenden werden die Funktion und konstruktive Details dieser bekannten CPAP-Vorrichtung näher erläutert.

Die Abmessungen betragen typischerweise:

| | |
|---|---|
| Länge Hohlzylinder | 15 mm |
| Durchmesser Hohlzylinder | 15 mm |
| Durchmesser Ring-Abstandshalter | 10 mm |
| Breite Ring-Abstandshalter | 10 mm |
| Länge Strömungsdüse | 15 mm, davon 5 mm im Ring-Abstandshalter |
| Durchmesser erste Öffnung | 5 mm |
| Durchmesser Sicherheits-Atemgasdurchtrittsöffnung | 3 mm |
| Länge Röhrenstummel | 5 mm |
| Durchmesser Röhrenstummel | 2 mm |
| Breite Nasenansatzstück-Quader | 15 mm |
| Höhe Nasenansatzstück-Quader | 5 mm |
| Tiefe Nasenansatzstück-Quader | 10 mm |
| Länge Laschen | 17 mm |

Die durch die Strömungsdüse 50 zugeführte Atemgasströmung beträgt typischerweise 10 bis 15 Liter pro Minute. Im zylindrischen Hohlraum 10 wird dadurch ein dazu proportionaler Überdruck aufgebaut. Je grösser die erste Öffnung 20 ist, desto geringer ist der aufgebaute Überdruck. Ziel ist es, eine genaue Balance zwischen der zugeführten Gasmenge, dem Zylindervolumen und dem Durchmesser der ersten Öffnung zu erreichen, so dass möglichst keine UmgebungsAtemgas während der Beatmung zugeführt wird, die die O₂-Konzentration verschieben könnte. Weiterhin sind auch die Ausgänge zum Nasenansatzstück optimiert, so dass ein möglichst kleiner Totraum bei der Atmung besteht.

Als Druckmessgerät kann im einfachsten Fall ein Wassersäulen-Messgerät mit Wasserschloss dienen, ansonsten sind natürlich auch aufwendigere elektronische Messgeräte anwendbar. Als Druckatemgas-Erzeugungsvorrichtung können alle üblichen Gastreibergeräte verwendet werden. Es sei bemerkt, dass durch die getroffene Auswahl von zwei Röhrenschnittstellen, einer für die Druckmessung und einer für die Gaszuführung, man in der Auswahl dieser beiden entsprechenden Geräte weitgehend freie Auswahl hat.

Eine wichtige Funktion hat die Sicherheits-Atemgasdurchtrittsöffnung am Ring-Abstandshalter. Wenn der Ring-Abstandshalter mit den Fingern beidseitig geschlossen wird, so resultiert eine unmittelbare Druckerhöhung. Diese wird zum Blähen der Lunge verwendet. Die Sicherheits-Atemgasdurchtrittsöffnung begrenzt jedoch den Druck auf einen vorbestimmten Wert, der insbesondere von deren Durchmesser abhängt. Wenn die CPAP-Vorrichtung mit den obigen Massen betrieben wird und der Druck bei Normalbetrieb 5 cm Wassersäule beträgt, so erreicht man durch seitliches Schliessen des Ring-Abstandshalters eine Erhöhung auf 12 cm Wassersäule.

Weiterhin ist es bekannt, dass bei der Neugeborenen-Atemluftbehandlung oft eine medikamentöse Zusatzbehandlung notwendig ist. Insofern sind separate Zusatzmasken mit vorgeschalteten Medikamentenverneblern bekannt. Als nachteilhaft bei diesem Ansatz hat sich die Tatsache herausgestellt, dass die Neugeborenen-Atemluftbehandlung zur medikamentösen Zusatzbehandlung unterbrochen werden muss.

Der Versuch, einen Vernebler zwischen die Druckluft-Erzeugungsvorrichtung und die Vorrichtung nach Fig. 3 zu schalten, führte zu unbefriedigenden Ergebnissen, u.a. da eine ungewünschte Kondensation aufgrund der ausgeatmeten Atemgasströmung B auftritt, die wärmer ist als die einzuatmende Atemgasströmung A.

Die der vorliegenden Erfindung zugrundeliegende Problematik besteht also allgemein darin, eine gattungsgemässe Vorrichtung zur Erzeugung eines kontinuierlichen positiven Atemwegdrucks (CPAP-Vorrichtung), und insbesondere eine nasale CPAP-Vorrichtung oder kurz nCPAP-Vorrichtung, derart weiterzubilden, dass die Neugeborenen-Atemluftbehandlung zur medikamentösen Zusatzbehandlung nicht unterbrochen werden muss.

### VORTEILE DER ERFINDUNG

Die erfindungsgemässe CPAP- bzw. nCPAP-Vorrichtung mit den Merkmalen des Anspruchs 1 weist gegenüber den bekannten Lösungsansätzen den Vorteil auf, dass die Neugeborenen-Atemluftbehandlung und die medikamentöse Zusatzbehandlung gleichzeitig synergetisch durchgeführt werden können, ohne dass es zu unerwünschten Kondensationseffekten kommt.

Die der vorliegenden Erfindung zugrundeliegende Idee besteht darin, dass eine zweite Öffnung in einer zweiten Seitenwand des Hohlkörpers zum Zuführen einer in den Hohlkörper gerichteten Medikamentenströmung vorgesehen ist.

In den Unteransprüchen finden sich vorteilhafte Weiterbildungen und Verbesserungen der in Anspruch 1 angegebenen nCPAP-Vorrichtung.

Gemäss einer bevorzugten Weiterbildung ist ein am Hohlkörper anbringbarer Abstandshalter vorgesehen, an dem eine Strömüngsdüse zum Richten der Atemgasströmung auf die erste Öffnung anbringbar ist.

Gemäss einer weiteren bevorzugten Weiterbildung weist der Hohlkörper im wesentlichen die Gestalt eines Hohlzylinders aufweist, an dessen einer Stirnfläche das Anschlussstück anbringbar ist und an dessen Mantelfläche die erste und zweite Öffnung vorgesehen sind.

Gemäss einer weiteren bevorzugten Weiterbildung liegen die erste und zweite Öffnung in etwas auf gleicher Höhe, so dass sich die in den Hohlkörper gerichtete Atemgasströmung und die in den Hohlkörper gerichtete Medikamentenströmung in einem Bereich zumindest teilweise überschneiden. Diese Anordnung bringt einen zusätzlichen Verwirbelungseffekt mit sich.

Gemäss einer weiteren bevorzugten Weiterbildung ist in die zweite Öffnung eine Röhre eingebracht, welche ins Innere des Hohlkörpers hineinragt.

Gemäss einer weiteren bevorzugten Weiterbildung ragt die Röhre so weit ins Innere des Hohlkörpers hineinragt, dass sie eine Abrisskante für die in den Hohlkörper gerichtete Atemgasströmung bildet. Diese Abrisskante sorgt für eine zusätzliche Verwirbelung.

Gemäss einer weiteren bevorzugten Weiterbildung ist die Röhre steckbar. Damit ist sie bei Bedarf leicht entfernbar bzw. anbringbar.

Gemäss einer weiteren bevorzugten Weiterbildung ist die zweite Öffnung durch eine Verschliesseinrichtung, vorzugsweise einen Deckel oder Schieber, verschliessbar.

Gemäss einer weiteren bevorzugten Weiterbildung sind die erste und zweite Öffnung in einem spitzen Winkel auf der Mantelfläche zueinander angeordnet.

Gemäss einer weiteren bevorzugten Weiterbildung besteht das Anschlussstück aus einem Stopfen, der an der Stirnfläche in den Hohlkörper zumindest teilweise einführbar ist. Vorzugsweise ist dabei keine der beiden Strömungen, Atemgasströmung uns Medikamentenströmung direkt auf das Anschlussstück gerichtet.

Gemäss einer weiteren bevorzugten Weiterbildung weist das Anschlussstück ein oder zwei Durchführungen auf, welche entsprechenden Atemgaswegen des Nasen- und/oder Mundansatzstücks entsprechen.

Gemäss einer weiteren bevorzugten Weiterbildung sind in den Durchführungen des Anschlussstücks ein oder zwei nach aussen weisende Röhrenstummel zum Einführen in das Nasenund/oder Mundansatzstück vorgesehen.

Gemäss einer weiteren bevorzugten Weiterbildung ist das Anschlussstück relativ zum Hohlkörper um mindestens eine bestimmte Achse verdrehbar ist und weist eine Verschliesseinrichtung auf, durch die die zweite Öffnung durch Drehen verschliessbar ist. Diese Verschliesseinrichtung könnte z.B. eine überstehende Lasche bzw. Klappe sein.

Gemäss einer weiteren bevorzugten Weiterbildung liegt die bestimmte Achse (Zylinderachse) im wesentlichen senkrecht zur Achse der in den Hohlkörper gerichteten Atemgasströmung und Medikamentenströmung. Somit ergibt sich ein Pralleffekt an der Zylinderwand, der die Durchmischung unterstützt.

Gemäss einer weiteren bevorzugten Weiterbildung weist der Abstandshalter eine im wesentlichen ringförmige oder becherförmige Gestalt auf.

Gemäss einer weiteren bevorzugten Weiterbildung ist die Strömungsdüse durch ein Loch in der Seitenwand der Ringgestalt bzw. Bechergestalt geführt und im wesentlichen senkrecht auf die erste Öffnung gerichtet.

Gemäss einer weiteren bevorzugten Weiterbildung ragt die Strömungsdüse um eine vorbestimmte Länge in das Ringgestaltinnere bzw. Bechergestaltinnere hinein.

Gemäss einer weiteren bevorzugten Weiterbildung ist in einer, vorzugsweise von der Seitenwand mit der ersten Öffnung verschiedenen Seitenwand des Hohlkörpers eine dritte Öffnung zum Anschliessen eines Druckmessgeräts vorgesehen.

### ZEICHNUNGEN

Ausführungsbeispiele der Erfindung sind in den Zeichnungen dargestellt und in der nachfolgenden Beschreibung näher erläutert.

Es zeigen:
- Fig. 1: eine schematische Darstellung einer Ausführungsform der erfindungsgemässen CPAP-Vorrichtung in einer ersten perspektivischen Ansicht;
- Fig. 2: eine schematische Darstellung der Ausführungsform der erfindungsgemässen CPAP-Vorrichtung in einer zweiten perspektivischen Ansicht;
- Fig. 3: eine schematische Darstellung einer aus der WO 99/24101 bekannten CPAP-Vorrichtung in perspektivischer Ansicht;
- Fig. 4: eine erste Querschnittsansicht eines Nasenansatzstücks zum Anschluss an das Ansatzstück von Fig. 3; und
- Fig. 5: eine zweite Querschnittsansicht des Nasenansatzstücks zum Anschluss an das Ansatzstück entlang der Linie A-A', die in Fig. 4 angedeutet ist.

### BESCHREIBUNG DER AUSFÜHRUNGSBEISPIELE

In den Figuren bezeichnen gleiche Bezugszeichen gleiche oder funktionsgleiche Elemente.

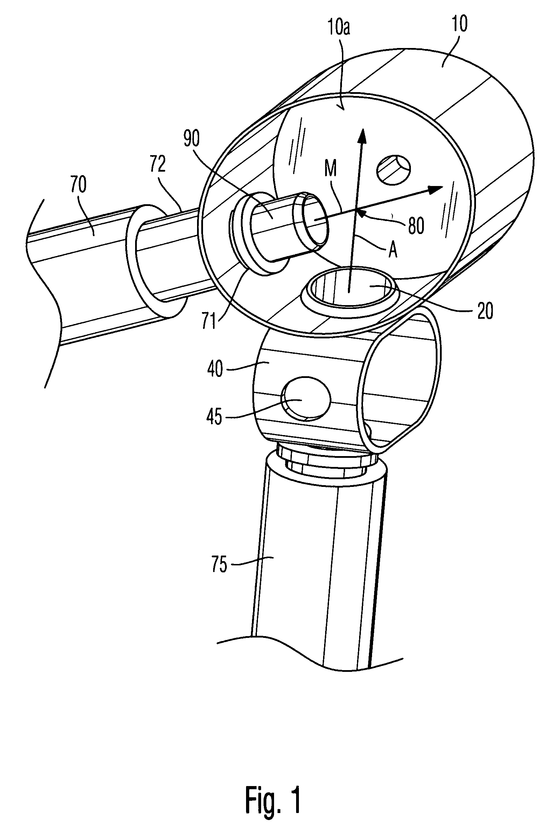

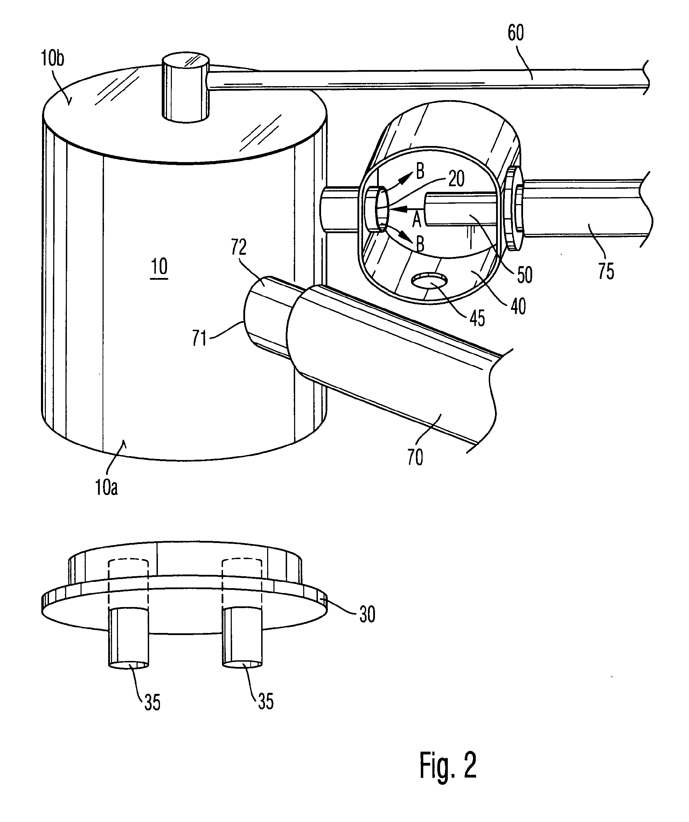

Fig. 1 zeigt eine schematische Darstellung einer Ausführungsform der erfindungsgemässen CPAP-Vorrichtung in einer ersten perspektivischen Ansicht und Fig. 2 in einer zweiten perspektivischen Ansicht.

Hauptbestandteile dieser Ausführungsform der erfindungsgemässen Vorrichtung zur Erzeugung eines kontinuierlichen positiven Atemwegdrucks sind diegleichen wie diejenigen, weiche einleitend in Bezug auf Fig. 3 bis 5 erläutert wurden.

Dies dargestellte Vorrichtung zur Erzeugung eines kontinuierlichen positiven Atemwegdrucks (CPAP-Vorrichtung), insbesondere nasale CPAP-Vorrichtung (nCPAP-Vorrichtung), umfasst also auch den Hohlkörper 10, in dem ein Überdruck aufbaubar ist.

In einer ersten Seitenwand des Hohlkörpers 10 ist die erste Öffnung 20 zum Zuführen einer in den Hohlkörper 10 gerichteten Atemgasströmung A und zum Abführen der ausgeatmeten Atemgasströmung B vorgesehen. Am Hohlkörper 10 anbringbar ist dassselbe Anschlussstück 30 zur Verbindung des Hohlkörpers 10 mit dem Nasen- und/oder Mundansatzstück 100.

Zusätzlich vorgesehen ist eine in einer zweiten Seitenwand des Hohlkörpers 10 vorgesehene zweite Öffnung 71 zum Zuführen einer in den Hohlkörper 10 gerichteten Medikamentenströmung M, welche z.B. von einem nicht gezeigten vorgeschalteten Einwegvernebler kommt.

An der Mantelfläche des Zylinders sind die erste und zweite Öffnung 20, 71 unter spitzem Winkel vorgesehen sind. Also ist bei diesem Beispiel die erste Seitenwand gleich der zweiten Seitenwand. Dies ist hinsichtlich des Vermischungseffekts vorteilhaft, muss jedoch nicht unbedingt der Fall sein.

Die erste und zweite Öffnung 20, 71 liegen in etwas auf gleicher Höhe, so dass sich die in den Hohlkörper 10 gerichtete Atemgasströmung A und die in den Hohlkörper 10 gerichtete Medikamentenströmung M in einem Bereich 80 überschneiden, bevor sie auf die Innenwand des Hohlzylinders treffen. Dadurch tritt eine vorteilhafte Nachverwirbelung bzw. -vernebelung auf.

In die zweite Öffnung 71 ist eine Röhre 90 entfernbar eingesteckt, welche ins Innere des Hohlkörpers 10 hineinragt und aussen in ein Ansatzstück 72 übergeht, auf das ein Schlauch 70 aufsteckbar ist.

Bei diesem Beispiel ragt die Röhre 90 so weit ins Innere des Hohlkörpers 10 hinein, dass sie eine Abrisskante für die in den Hohlkörper 10 gerichtete Atemgasströmung A bildet. Dies ist nicht unbedingt notwendig, unterstützt aber den Nachverwirbelungseffekt.

Obwohl nicht gezeigt, ist die zweite Öffnung 71 durch eine Verschliesseinrichtung in Form von einem Deckel verschliessbar. Somit der Medikamentenport bei Bedarf verschliessbar bzw. anschliessbar.

Im Betrieb lassen sich durch diesen Aufbau die Neugeborenen-Atemluftbehandlung und die medikamentöse Zusatzbehandlung gleichzeitig synergetisch durchführen, ohne dass es zu unerwünschten Kondensationseffekten kommt. Bei fehlendem Bedarf nach medikamentöser Zusatzbehandlung wird die Röhre 90 einfach ausgesteckt und der Hohlkörper verschlossen.

Obwohl die vorliegende Erfindung anhand eines bevorzugten Ausführungsbeispiels vorstehend beschrieben wurde, ist sie darauf nicht beschränkt, sondern auf vielfältige Weise modifizierbar.

Die Verschliessfunktion könnte auch durch einen im Innern oder Äusseren des Hohlkörpers 10 angebrachten Schieber realisiert werden. Auch könnte das Anschlussstück 30, das relativ zum Hohlkörper 10 um die Zylinderachse verdrehbar ist und eine Verschliesseinrichtung in Form einer Verlängerung am unteren Umfand aufweisen, durch die die zweite Öffnung 71 durch Drehen verschliessbar ist.

Insbesondere können die Dimensionen den jeweiligen Anforderungen entsprechend gewählt werden. Auch kann der Hohlraum eine beliebige andere Form als die Zylinderform aufweisen.

## Patentansprüche

1. Vorrichtung zur Erzeugung eines kontinuierlichen positiven Atemwegdrucks (CPAP-Vorrichtung), insbesondere nasale CPAP-Vorrichtung (nCPAP-Vorrichtung), mit:
einem Hohlkörper (10), in dem ein Überdruck aufbaubar ist;
einer in einer ersten Seitenwand des Hohlkörpers (10) vorgesehenen ersten Öffnung (20) zum Zuführen einer in den Hohlkörper (10) gerichteten Atemgasströmung (A) und zum Abführen der ausgeatmeten Atemgasströmung (B); und
einem am Hohlkörper (10) anbringbaren Anschlussstück (30) zur Verbindung des Hohlkörpers (10) mit einem Nasenund/oder Mundansatzstück (100);
**gekennzeichnet durch**
eine in einer zweiten Seitenwand des Hohlkörpers (10) vorgesehene zweite Öffnung (71) zum Zuführen einer in den Hohlkörper (10) gerichteten Medikamentenströmung (M).

2. Vorrichtung nach Anspruch 1, **dadurch gekennzeichnet, dass** ein am Hohlkörper (10) anbringbarer Abstandshalter (40) vorgesehen ist, an dem eine Strömungsdüse (50) zum Richten der Atemgasströmung (A) auf die erste Öffnung (20) anbringbar ist.

3. Vorrichtung nach Anspruch 1 oder 2, **dadurch gekennzeichnet, dass** der Hohlkörper (10) im wesentlichen die Gestalt eines Hohlzylinders aufweist, an dessen einer Stirnfläche (10a) das Anschlussstück (30) anbringbar ist und an dessen Mantelfläche die erste und zweite Öffnung (20; 71) vorgesehen sind.

4. Vorrichtung nach Anspruch 3, **dadurch gekennzeichnet, dass** die erste und zweite Öffnung (20; 71) in etwas auf gleicher Höhe liegen, so dass sich die in den Hohlkörper (10) gerichtete Atemgasströmung (A) und die in den Hohlkörper (10) gerichtete Medikamentenströmung (M) in einem Bereich (80) zumindest teilweise überschneiden.

5. Vorrichtung nach einem der vorhergehenden Ansprüche, **dadurch gekennzeichnet, dass** in die zweite Öffnung (71) eine Röhre (90) eingebracht ist, welche ins Innere des Hohlkörpers (10) hineinragt.

6. Vorrichtung nach Anspruch 5, **dadurch gekennzeichnet, dass** die Röhre (90) so weit ins Innere des Hohlkörpers (10) hineinragt, dass sie eine Abrisskante für die in den Hohlkörper (10) gerichtete Atemgasströmung (A) bildet.

7. Vorrichtung nach Anspruch 5 oder 6, **dadurch gekennzeichnet, dass** die Röhre (90) steckbar ist.

8. Vorrichtung nach einem der vorhergehenden Ansprüche, **dadurch gekennzeichnet, dass** die zweite Öffnung (71) durch eine. Verschliesseinrichtung, vorzugsweise einen Deckel oder Schieber, verschliessbar ist.

9. Vorrichtung nach einem der vorhergehenden Ansprüche 3 bis 8, **dadurch gekennzeichnet, dass** die erste und zweite Öffnung (20; 71) in einem spitzen Winkel auf der Mantelfläche zueinander angeordnet sind.

10. Vorrichtung nach einem der vorhergehenden Ansprüche 3 bis 9, **dadurch gekennzeichnet, dass** das Anschlussstück (30) aus einem Stopfen besteht, der an der Stirnfläche (10a in den Hohlkörper (10) zumindest teilweise einführbar ist.

11. Vorrichtung nach einem der vorhergehenden Ansprüche, **dadurch gekennzeichnet, dass** das Anschlussstück (30) ein oder zwei Durchführungen aufweist, welche entsprechenden AtemAtemgaswegen des Nasen- und/oder Mundansatzstücks (100) entsprechen.

12. Vorrichtung nach Anspruch 11, **dadurch gekennzeichnet, dass** in den Durchführungen des Anschlussstücks (30) ein oder zwei nach aussen weisende Röhrenstummel (35) zum Einführen in das Nasen- und/oder Mundansatzstück (100) vorgesehen sind.

13. Vorrichtung nach einem der vorhergehenden Ansprüche, **dadurch gekennzeichnet, dass** das Anschlussstück (30) relativ zum Hohlkörper (10) um mindestens eine bestimmte Achse verdrehbar ist und eine Verschliesseinrichtung aufweist, durch die die zweite Öffnung (71) durch Drehen verschliessbar ist.

14. Vorrichtung nach Anspruch 13, **dadurch gekennzeichnet, dass** die bestimmte Achse im wesentlichen senkrecht zur Achse der in den Hohlkörper (10) gerichteten Atemgasströmung (A) und Medikamentenströmung (M) liegt.

15. Vorrichtung nach einem der vorhergehenden Ansprüche 2 bis 14, **dadurch gekennzeichnet, dass** der Abstandshalter (40) eine im wesentlichen ringförmige oder becherförmige Gestalt aufweist.

16. Vorrichtung nach Anspruch 15, **dadurch gekennzeichnet, dass** die Strömungsdüse (50) durch ein Loch in der Seitenwand der Ringgestalt bzw. Bechergestalt geführt ist und im wesentlichen senkrecht auf die erste Öffnung (20) gerichtet ist.

17. Vorrichtung nach Anspruch 16, **dadurch gekennzeichnet, dass** die Strömungsdüse (50) um eine vorbestimmte Länge in das Ringgestaltinnere bzw. Bechergestaltinnere hineinragt.

18. Vorrichtung nach einem der vorhergehenden Ansprüche, **dadurch gekennzeichnet, dass** in einer, vorzugsweise von der Seitenwand mit der ersten Öffnung (20) verschiedenen Seitenwand des Hohlkörpers (10) eine dritte Öffnung zum Anschliessen eines Druckmessgeräts vorgesehen ist.
